# EUROPEAN PATENT APPLICATION

(11) **EP 4 847 609 A1**
(43) Date of publication of application: **02.12.2026**
(21) Application number: 25758673.5
(22) Date of filing: 24.02.2025
(51) Int. Cl.: C12N 9/16, C12N 15/70, A23K 20/189, A62D 3/02

(54) **NOVEL POLYPEPTIDE WITH ZEARALENONE-DEGRADING ACTIVITY**

(30) Priority: 23.02.2024 KR 20240026536
(71) Applicant: CJ Cheiljedang Corporation, Seoul 04560 (KR)
(72) Inventor: YANG, Tae Joo, Seoul 04560 (KR); LEE, Seung-hee, Seoul 04560 (KR); PARK, Taeseo, Seoul 04560 (KR); KANG, Young Seo, Seoul 04560 (KR); KIM, Nam Yoon, Seoul 04560 (KR); LEE, Whiso, Seoul 04560 (KR)
(74) Representative: Meissner Bolte Partnerschaft mbB
(86) International application number: PCT/KR2025/002569
(87) International publication number: WO 2025/178424

(57) **Abstract**

The present disclosure relates to a variant polypeptide having ZEN-degrading activity and uses thereof.

## Description

### [Technical Field]

The present disclosure relates to a variant polypeptide having zearalenone-degrading activity and uses thereof.

### [Background Art]

Mycotoxins are substances primarily secreted by *Aspergillus*, *Fusarium*, and *Penicillium*, and cause various diseases, including cancer, miscarriage, and stillbirth, upon ingestion by humans and livestock.

Among the widely known mycotoxins, zearalenone (ZEN) is a mycotoxin produced by fungi of the genus *Fusarium*. ZEN is a non-steroidal estrogenic mycotoxin, also referred to as F-2 mycotoxin or fermentation estrogenic substance (FES). It has a chemical structure and properties similar to the female hormone estrogen and thus may cause endocrine disruption in humans and livestock. For example, a recent study reported that receptors damaged by ZEN suppress the hormone estrogen in mouse mammary tissues (Ecotoxicology and Environmental Safety Volume 241, August 2022, 113826). In addition, it was reported that ZEN stimulates the growth of human breast cancer cells with estrogen-responsive receptors (Toxins 2019, 11(8), 481). Prolonged ingestion of cereals contaminated with zearalenone may induce infertility in pigs, and poisoning in humans and livestock can cause cancers or mutations. Accordingly, there is a need for a composition or method for effectively degrading and detoxifying zearalenone.

Meanwhile, one important consideration in the development of a feed enzyme is the acid stability of the enzyme. This is due to the unique environment of the stomach, a digestive organ. When food enters the stomach, hydrochloric acid is secreted to reduce the food to a pulp-like consistency and to control harmful bacteria. In particular, the intragastric pH is maintained at a pH level of approximately 2.0 to 3.0, and if acid stability is insufficient, the enzyme is inactivated during the transit time through the stomach and becomes unable to act in the intestine. Accordingly, the acid stability of the enzyme is important for the enzyme to be active in the intestine.

Therefore, there is a need for the development of an effective degrading enzyme that effectively degrades zearalenone and is active in the intestine.

### [Disclosure]

### [Technical Problem]

The object to be solved by the present disclosure is to provide: a variant polypeptide having zearalenone (ZEN)-degrading activity; a polynucleotide encoding the variant polypeptide; a host cell comprising at least one selected from the variant polypeptide and a polynucleotide encoding the polypeptide; a composition for degrading zearalenone, comprising at least one selected from the variant polypeptide and a host cell expressing the variant polypeptide; a composition for use as a feed additive; a method for degrading zearalenone, comprising contacting zearalenone with at least one selected from the variant polypeptide and a host cell expressing the variant polypeptide; and a method for preparing the variant polypeptide having zearalenone-degrading activity.

### [Technical Solution]

One object of the present disclosure is to provide a variant polypeptide having zearalenone (ZEN)-degrading activity.

Another object of the present disclosure is to provide a polynucleotide encoding the variant polypeptide of the present disclosure.

Still another object of the present disclosure is to provide a host cell comprising at least one selected from the variant polypeptide of the present disclosure and a polynucleotide encoding the variant polypeptide.

Still another object of the present disclosure is to provide a composition for degrading ZEN, comprising at least one selected from the variant polypeptide of the present disclosure and a host cell comprising the variant polypeptide.

Still another object of the present disclosure is to provide a composition for use as a feed additive, comprising at least one selected from the variant polypeptide of the present disclosure and a host cell comprising the variant polypeptide.

Still another object of the present disclosure is to provide a method for degrading ZEN, comprising contacting ZEN with at least one selected from the variant polypeptide of the present disclosure and a host cell comprising the variant polypeptide.

Still another object of the present disclosure is to provide a method for preparing a variant polypeptide having ZEN-degrading activity, comprising: culturing a host cell comprising at least one selected from the variant polypeptide of the present disclosure and a polynucleotide encoding the polypeptide; and recovering the variant polypeptide having ZEN-degrading activity expressed during the culturing.

### [Advantageous Effects]

The variant polypeptide having zearalenone (ZEN)-degrading activity of the present disclosure exhibits improved acid stability, thereby allowing effective degradation of zearalenone, a mycotoxin, even under pH-varying conditions.

### [Brief Description of the Drawings]

FIG. 1 is a diagram showing the results of confirming the zearalenone (ZEN)-degrading activity of the variant polypeptide of the present disclosure using HPLC-UV.
FIG. 2 is a diagram showing the results of evaluating the enzyme acid stability of the variant polypeptide of the present disclosure.

### [Detailed Description of Preferred Embodiments]

The present disclosure will be described in detail as follows. Meanwhile, each description and embodiment described herein may be applied to other descriptions and embodiments, respectively. That is, all combinations of various elements disclosed herein fall within the scope of the present disclosure. In addition, the scope of the present disclosure is not limited to the specific description described below.

Furthermore, those skilled in the art may recognize or identify numerous equivalents to the specific aspects of the present disclosure described herein by using routine experimentation. Moreover, these equivalents are intended to be comprised in the present disclosure.

As used in the specification and appended claims of the present disclosure, the singular articles ("a", "an", and "the") comprise plural referents unless the context clearly indicates otherwise. In addition, unless otherwise indicated by the context, singular terms comprise their plural forms, and plural terms comprise their singular forms. As used in the specification and appended claims of the present disclosure, the term "or" may comprise the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. As another example, the term "about" may refer to a range of -5% to +5% of the given numerical value. However, the term is not limited thereto.

As used herein, terms such as "first, second, third,...", "i), ii), iii),...", or "(a), (b), (c), (d),..." are used to distinguish similar elements and do not imply that the elements are executed continuously or in the listed order. For example, when these terms are used in relation to steps of a method, use, or analysis, the steps may be performed without any time intervals, simultaneously, or with intervals of several seconds, minutes, hours, days, or months.

As used herein, the term "consisting essentially of" may mean that, in the case where the features of a matter claimed herein are substantially unaffected by the presence of an unspecified component, the unspecified component may be present.

As used herein, the term "consisting of" means that the ratio of the specific component(s) sums up to 100%. The components or features recited after the term "consisting of" may be essential or mandatory. In some specific embodiments, in addition to the components or features recited after the term "consisting of", other arbitrary or non-essential components may be excluded.

As used herein, the term "comprising" refers to the presence of the features, steps, or components following the term and does not exclude the presence or addition of at least one other feature, step, or component. As used herein, the components or features following the term "comprising" may be essential or mandatory; however, in certain specific embodiments, other optional or non-essential components or features may further be comprised.

As used herein, in certain specific embodiments, the term "comprising" may be modified to refer to "consisting essentially of" or "consisting of".

With respect to amino acid sequences in the present disclosure, even if it is disclosed as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that a protein having an amino acid sequence in which a portion of the sequence is deleted, modified, substituted, conservatively substituted, or added can be used in the present disclosure if it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution, but is not limited thereto.

As used herein, the term "protein" or "polypeptide" refers to a polymer or oligomer of consecutive amino acid residues. As used herein, "polypeptide", "protein", and "peptide" may be used interchangeably with "amino acid sequence".

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, nucleic acid, polypeptide, or vector, means that the cell, nucleic acid, polypeptide, or vector has been modified by the introduction of a heterologous nucleic acid or polypeptide, or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, under-expressed, or not expressed at all.

As used herein, the term "isolated" refers to a substance that exists in a non-naturally occurring environment or does not exist naturally. It comprises at least substantially isolating a substance (for example, a sequence, enzyme, or nucleic acid) from a substance that is naturally associated and found in nature, for example, at least one other component having the substance (sequence, enzyme, or nucleic acid).

For example, the isolated sequence, enzyme, or nucleic acid provided herein may be provided in a form that is substantially free of one or more contaminants.

Examples of the isolated substance may comprise: i) any non-naturally occurring substance; ii) any substance (for example, enzyme, variant, nucleic acid, protein, peptide, or cofactor) from which one, or more, or all naturally occurring components associated in nature have been removed; iii) any substance that has been artificially modified from a substance found in nature; or iv) a substance that has been modified (*e.g.*, increase in copy number of a gene encoding a specific substance; modification of a promoter naturally linked to a gene encoding a specific substance into a highly active promoter, *etc.*) to have altered amount of a substance relative to other components associated in nature, but are not limited thereto.

As used herein, the term "wild-type" refers to a naturally occurring state without artificial modification. When the term "wild-type" is used in reference to a polypeptide, it refers to a naturally occurring polypeptide which does not have artificial mutation (substitution, insertion, deletion, *etc.*) at at least one amino acid position. Similarly, when the term "wild-type" is used in reference to a polynucleotide, it refers to a polynucleotide lacking artificial modification (substitution, insertion, deletion) of at least one nucleotide. However, a polynucleotide encoding a wild-type polypeptide is not limited to native polynucleotides and also comprises sequences encoding an arbitrary wild-type polypeptide.

As used herein, the term "parent sequence" or "backbone" refers to a reference sequence in which modification is introduced to create a modified polypeptide. That is, the parent sequence may be a starting sequence into which modifications such as substitutions, additions, and/or deletions are to be introduced. The parent sequence may be naturally occurring or wild-type, or a variant in which one or more substitutions, insertions, or deletions have occurred in the natural or wild type, or may be an artificially synthesized sequence. When the parent sequence is an amino acid sequence exhibiting an activity, *i.e.*, an amino acid sequence of an enzyme, it may be referred to as a parent enzyme.

As used herein, the term "reference sequence" refers to a sequence used to determine the position of amino acids within any amino acid sequence. By aligning an arbitrary amino acid sequence with a reference sequence, the amino acid position in the arbitrary sequence that corresponds to a specific position in the reference sequence can be determined.

As used herein, in reference to amino acid or nucleic acid sequences, the term "fragment" refers to a portion of a parent sequence. For example, it may be a polypeptide in the form in which one or more amino acids are removed from the C-terminus or N-terminus of the parent sequence.

As used herein, the "fragment" of an enzyme may refer to a "functional fragment". The term "functional fragment" may also be referred to as an active fragment, and refers to a polypeptide that is a portion of a parent enzyme and has enzymatic activity of the parent enzyme. For example, the functional fragment of an enzyme may comprise a catalytic site of the enzyme.

A fragment of the enzyme may comprise a portion of the full length of the parent enzyme. For example, it may comprise amino acids of at least about 20%, 30%, 40%, 50%, 60%, 70%, 80%, 90%, 95%, 98%, 99% or more, and less than 100% of the full length of the parent enzyme, but is not limited thereto.

In the present disclosure, "modifying" means changing or altering. It may be altering from a naturally occurring state. In one example, an enzyme may be altered in a manner that causes it to differ from a parent sequence or a reference sequence.

In the present disclosure, a modified enzyme may be an enzyme that does not exist in nature by itself, *i.e.*, a non-naturally occurring enzyme.

As used herein, the term "modified" refers, for example, to something that has been altered from its naturally occurring state. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, when the term "modification" is used in relation to an amino acid/nucleic acid sequence, it may comprise a substitution of an amino acid/nucleic acid residue of the parent sequence with a different amino acid/nucleic acid residue at at least one portion in the amino acid sequence, a deletion of an amino acid/nucleic acid residue (or a series of amino acid/nucleic acid residues) of the parent sequence at at least one position, an insertion of an amino acid/nucleic acid residue (or a series of amino acid/nucleic acid residues) of the parent sequence at at least one position, a truncation of the amino acid sequence at the N-terminus and/or C-terminus or the nucleic acid sequence at the 5' and/or 3' end, and any combination thereof.

As used herein, the "variant" or "modified polypeptide" of an enzyme refers to a protein having at least one amino acid different from that of a parent enzyme by conservative substitution and/or modification. The "variant" or "modified polypeptide" may be used interchangeably. The variant or modified polypeptide may be non-naturally occurring, but is not limited thereto.

The variant differs from the sequence of the parent enzyme by at least one modification, for example, substitution, deletion, and/or insertion of amino acids.

Such a variant may generally be identified by modifying at least one amino acid in the parent enzyme and evaluating the properties of the modified protein. That is, the ability of a variant may be enhanced, unchanged, or reduced relative to the parent enzyme.

Additionally, certain variants may comprise a variant polypeptide in which at least one region, such as an N-terminal leader sequence or transmembrane domain, has been removed.

Other variants may comprise a variant in which a portion has been removed from the N- and/or C-terminus of the mature protein.

The term "variant" or "variant polypeptide" may be used interchangeably with terms comprising modification, modified protein, mutant, mutein, divergent, and variant, and is not limited as long as the terms are used to indicate mutation.

The variant may also comprise the deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. In addition, the polypeptide may also be conjugated with another sequence or linker to allow for identification, purification, or synthesis of the polypeptide.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues.

Throughout the entire specification of the present disclosure, the conventional one-letter and three-letter codes for naturally occurring amino acids are used. Additionally, the amino acids mentioned herein are abbreviated according to the IUPAC-IUB nomenclature as follows:

| | |
|---|---|
| alanine Ala, A | arginine Arg, R |
| asparagine Asn, N | aspartate Asp, D |
| cysteine Cys, C | glutamate Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa or X.

In addition to naturally occurring amino acids, commonly accepted three-letter codes may also be used for other amino acids such as 2-aminoisobutyric acid (Aib), N-methylglycine (Sar), and α-methyl-glutamate.

Amino acids can generally be classified based on similarity in polarity, charge, solubility, hydrophobicity, hydrophilicity, and/or amphipathic nature of the residues. Accordingly, amino acid substitution may generally occur based on similarity in polarity, charge, solubility, hydrophobicity, hydrophilicity, and/or amphipathic nature of the residues.

For example, among the amino acids having an electrically charged side chain (electrically charged amino acids), positively charged (basic) amino acids comprise arginine, lysine, and histidine, and negatively charged (acidic) amino acids comprise glutamate and aspartate. In addition, among the amino acids having an uncharged side chain (uncharged amino acids), nonpolar amino acids comprise glycine, alanine, valine, leucine, isoleucine, methionine, phenylalanine, tryptophan, and proline, and polar or hydrophilic amino acids comprise serine, threonine, cysteine, tyrosine, asparagine, and glutamine. Among the nonpolar amino acids, aromatic amino acids comprise phenylalanine, tryptophan, and tyrosine.

As used herein, the term "gene" means a polynucleotide that encodes a polypeptide and a polynucleotide comprising the upstream and downstream regions of the coding region. In certain specific embodiments, a gene may have a sequence (intron) inserted between each coding region (exon).

As used herein, the term "homology" or "identity" refers to the degree of relevance between two given amino acid sequences or nucleotide sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length sequence or to at least about 50%, 60%, 70%, 80%, or 90% or more of the full-length. It is apparent that hybridization also comprises hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are comprised). For example, homology, similarity, or identity may be determined using BLAST from the National Center for Biotechnology Information (NCBI) or ClustalW, but is not limited thereto.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program may be defined as the value obtained by dividing the number of similarly aligned symbols (*i.e.*, nucleotides or amino acids) by the total number of symbols in the shorter of the two sequences. The default parameters for the GAP program may comprise: (1) a binary comparison matrix (containing a value of 1 for identity and 0 for non- identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In addition, whether any two polynucleotide or polypeptide sequences have homology, similarity, or identity may be identified by comparing the sequences in a Southern hybridization experiment under stringent conditions as defined. Appropriate hybridization conditions defined are within the skill of the art, and may be determined by a method well known to those skilled in the art (*e.g.*, J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the method for identifying homology, similarity, or identity and the appropriate hybridization conditions are not limited thereto.

As used herein, the term "mature polypeptide" refers to a polypeptide in a form without a signal sequence or propeptide sequence. A mature protein/polypeptide/peptide may be a functional form of a protein/polypeptide/peptide. A mature polypeptide may be in a final form after translation or post-translational modification. Examples of post-translational modification comprise N-terminal or C-terminal modifications, glycosylation, phosphorylation, removal of a leader sequence, *etc.*, but are not limited thereto.

As used herein, the term "nucleic acid construct" refers to a single- stranded or double-stranded nucleic acid molecule that comprises at least one regulatory sequence and is artificially synthesized, engineered to comprise a specific sequence in a non-naturally occurring manner, or isolated from nature.

As used herein, the term "expression" comprises an arbitrary step involved in polypeptide production, for example, transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression.

As used herein, the term "operably linked" refers to a constitution in which a regulatory sequence is positioned appropriately to initiate the expression of a coding sequence. Hence, "operably linked" comprises the attachment or linkage of a regulatory region of a functional domain with a known or desired activity, such as a promoter, terminator, signal sequence, or enhancer region, to a target (gene or polypeptide) to regulate its expression, secretion, or function in accordance with the known or desired activity.

As used herein, the term "cDNA" refers to a DNA sequence that can be produced by reverse transcription from a mature, spliced mRNA molecule obtainable from a eukaryotic or prokaryotic cell. A cDNA sequence does not comprise intron sequences that may be present in the corresponding genomic DNA. An initial primary RNA transcript is a precursor to mRNA that undergoes a series of steps comprising splicing to become mature spliced mRNA.

As used herein, the term "regulatory sequence" refers to a polynucleotide sequence necessary for the expression of a coding sequence. Each regulatory sequence may be native (derived from the same origin) or foreign (derived from a different gene) to the coding sequence. Examples of the regulatory sequence comprise a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may comprise a promoter and a sequence for terminating transcription and translation.

In order to describe the variant provided in the present disclosure, the following nomenclature is used.

In the present disclosure, referring to a specific position in an amino acid sequence may comprise referring to an amino acid present or substituted at that position. Referring to an amino acid at a specific position can be described in various ways. For example, "position 022" may be disclosed as "position 22", "amino acid at position 22", and "22^{nd} amino acid". In addition, for example, when the amino acid at position 22 is proline (P), the amino acid may be disclosed as "P22" or "Pro22".

Amino acid substitution may be expressed by describing, in order, the amino acid before substitution, the position, and the substituted amino acid. The amino acids may be expressed using the conventional one-letter and three-letter codes. In one example, when the amino acid proline at position 22 in a specific sequence is substituted with threonine, it may be disclosed as "P22T" or "Pro22Thr".

Any amino acid at a specific position may be referred to as "X". For example, X22 refers to any amino acid at position 22. Additionally, when the substituted amino acid is expressed as X, it means that it is substituted with an amino acid different from the amino acid before substitution. For example, "P22X" refers to the substitution of P at position 22 with an arbitrary amino acid that is not P.

Different alterations can be expressed by simultaneously describing several types of amino acids using the symbol "/" or ",". For example, the substitution of the amino acid (K) at position 43 with A or C may be interchangeably disclosed as K43A/C or K43A,C. In another example, A/T111C means that the amino acid A or T at position 111 before substitution is substituted with C.

Multiple mutations can be described using "+". For example, descriptions such as "A45C+T111C" mean that the amino acid alanine at position 45 and the amino acid threonine at position 111 are each substituted with cysteine.

The deletion of an amino acid can be expressed by describing, in order, the amino acid before deletion, the position, and *. For example, when the amino acid alanine corresponding to position 45 in a specific sequence is deleted, it may be expressed as A45* or (Ala45*).

The insertion of an amino acid may be expressed, for example, as Gly8GlyLys or G8GK in the case where lysine is inserted between glycine at position 8 and the amino acid at position 9 in a specific sequence. When at least one amino acid is inserted, for example, in the case where lysine and valine are inserted between glycine at position 8 and the amino acid at position 9 in a specific sequence, it may be expressed as Gly8GlyLysVal or G8GKV. In such a case, the position of the inserted amino acid may be indicated using the number and alphabet of the amino acid preceding the inserted amino acid, and taking the above-described case as an example, the inserted lysine and valine may be numbered as 8aK and 8bV.

As used herein, the term "corresponding to" refers to an amino acid residue at the recited position in a protein or peptide, or an amino acid residue that is similar, identical, or homologous to the recited residue in a protein or peptide. Identification of the amino acid at the corresponding position may be determining a specific amino acid in a sequence that references a particular sequence. As used herein, the term "corresponding region" refers to a similar or corresponding position in a related protein or a reference protein.

In the present disclosure, SEQ ID NO: 1 can be used as a reference sequence to determine the position of amino acids in any amino acid sequence.

That is, SEQ ID NO: 1 disclosed herein can be used to determine the corresponding amino acid residues in a polypeptide having ZEN-degrading activity. Unless indicated otherwise in the present disclosure, residues of a specific amino acid sequence are numbered relative to SEQ ID NO: 1.

For example, based on the alignment of an arbitrary amino acid sequence with SEQ ID NO: 1, each amino acid residue in the amino acid sequence can be numbered with reference to the position number of the amino acid residue corresponding to the amino acid residue of SEQ ID NO: 1. For example, a sequence alignment algorithm as described herein can confirm the position of an amino acid or a position where modification such as substitution, insertion, or deletion occurs compared to a query sequence (also referred to as the "reference sequence").

For such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), and the like can be used, but are not limited thereto.

Additionally, multiple sequence alignment can be used to identify corresponding amino acid residues in other ZEN-degrading enzymes. Examples of multiple sequence alignment programs known in the art comprise multiple sequence comparison by log-expectation (MUSCLE; version 3.5 or higher; Edgar, 2004, Nucleic Acids Research 32: 1792-1797), MAFFT (version 6.857 or higher; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), EMBOSS EMMA using ClustalW (1.83 or higher; Thompson et al., 1994, Nucleic Acids Research 22 : 4673-4680), and the like, and the basic parameters of each of the programs may be used, but are not limited thereto.

In addition, in the case where the relationship between the enzymes branched from the mature polypeptide of SEQ ID NO: 1 cannot be detected by conventional sequence-based comparison, other pairwise sequence comparison algorithms may be used (Lindahl and Elofsson, 2000, J. Mol. Biol. 295: 613-615). Higher sensitivity may be achieved in sequence-based searches by using search programs that utilize probabilistic representations of polypeptide families (profiles) to search databases. For example, the PSI-BLAST program can generate a profile through an iterative database search process and detect remote homologs (Atschul et al., 1997, Nucleic Acids Res. 25: 3389-3402). When the family or superfamily of a polypeptide has one or more representatives in protein structure databases, significantly higher sensitivity may be achieved. Programs such as GenTHREADER (Jones, 1999, J. Mol. Biol. 287: 797-815; McGuffin and Jones, 2003, Bioinformatics 19: 874-881) use information from various sources, such as PSI-BLAST, secondary structure prediction, structural alignment profiles, and solvation potentials, as input to neural networks that predict structural folding for query sequences. Similarly, the method of Gough et al., 2000, J. Mol. Biol. 313: 903-919 may be used to align sequences of unknown structure with superfamily models existing in the SCOP database. These alignments may be sequentially used to generate homology models for the peptides, and such models can be assessed for accuracy using a variety of tools developed for the same purpose.

For proteins of known structure, several tools and resources may be used for searching and generating structural alignments. For example, the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms may additionally be utilized to query structure databases with a structure of interest in order to discover possible structural homologs (Holm and Park, 2000, Bioinformatics 16: 566-567).

The above methods are illustrative examples and are not limited thereto.

Hereinafter, the specific embodiments of the present disclosure will be described in more detail.

As used herein, "zearalenone (ZEN)" refers to a non-steroidal estrogenic mycotoxin produced by fungi of the genus *Fusarium*, in particular strains such as *F. graminearum* (*Gibberella zeae*), *F. culmorum*, *F. cerealis*, *F. equiseti*, *F. crookwellense*, and *F. semitectum*, and is known as one of the mycotoxins widely distributed in grains worldwide. ZEN is also referred to as F-2 toxin, Fusarium toxin, and fermentation estrogenic substance (FES), and has more than 20 isomers, but trans-α-zearalenone is the one predominantly found in grains in nature. ZEN has been reported to cause damage primarily through actions similar to sex hormones rather than through direct toxicity, and intoxication is mostly manifested as hyperhormonalism, and may also be manifested as cancer or mutation. For example, ZEN has been reported to cause infertility in pregnant sows in the livestock industry, and ovarian abnormalities, premature birth, and miscarriage have been reported. It is known that feed containing ZEN at a concentration of 50 ppb can induce changes in the ovaries of pigs.

As used herein, a ZEN-degrading enzyme refers to an enzyme having an activity of cleaving the lactone ring ester bond of zearalenone (ZEN), and catalyzes a reaction that converts ZEN into hydrolyzed ZEN (HZEN).

In the present disclosure, the degradation of ZEN may be used interchangeably with the detoxification of ZEN, the inactivation of ZEN, and the decontamination of ZEN.

In the present disclosure, ZEN-degrading activity can be measured and evaluated by using methods known in the art, comprising embodiments described in the present disclosure. In one example, the ZEN-degrading activity can be confirmed by detecting the residual amount of zearalenone.

As used herein, the term "parent ZEN-degrading enzyme" refers to a ZEN-degrading enzyme that is modified to produce the variant or variant polypeptide of the present disclosure. Specifically, the parent ZEN-degrading enzyme, parent enzyme, or parent sequence may be a naturally occurring or wild-type polypeptide or a mature polypeptide thereof, and may comprise a variant or functional fragment thereof. However, it is not limited thereto, as long as it is a polypeptide that has ZEN-degrading activity and is capable of serving as a parent of a variant.

The parent ZEN-degrading enzyme provided herein may be the polypeptide of SEQ ID NO: 1, but is not limited thereto. In addition, the parent ZEN-degrading enzyme may be a polypeptide having at least about 85%, 90%, 95%, 96%, 97%, 98%, or 99% sequence identity to the polypeptide of SEQ ID NO: 1 as long as it exhibits ZEN-degrading activity, and a polypeptide may be comprised in the scope of the parent ZEN-degrading enzyme without limitation if it has the same or equivalent activity as the polypeptide consisting of the amino acid sequence of SEQ ID NO: 1.

The parent ZEN-degrading enzyme of the variant provided herein may be derived from the genus *Sphingomonas*.

Meanwhile, the above-described microorganism is an example of a microorganism from which the parent ZEN-degrading enzyme provided herein may be derived, and comprises microorganisms derived from taxonomically homologous microorganisms regardless of the name of the microorganism.

The above-described microorganism can be obtained from known microorganism deposit institutions such as ATCC, DSMZ, CBS, NRRL, KCTC, and KCCM.

In the present disclosure, a sequence "derived from" a specific microorganism is not limited to those naturally produced or producible in the microorganism, but also comprises sequences encoded by genes produced and isolated from microorganisms comprising the gene.

For example, a ZEN-degrading enzyme derived from the genus *Sphingomonas* comprises not only an enzyme having ZEN-degrading activity that is naturally produced in *Sphingomonas*, but also one produced from a *Sphingomonas* source, and one produced in other host cells through genetic modification known in the art (for example, transformation with a sequence encoding the enzyme).

As used herein, the "variant polypeptide having ZEN-degrading activity" may be a variant of a parent ZEN-degrading enzyme.

As used herein, the term "variant of a parent ZEN-degrading enzyme" or "ZEN-degrading enzyme variant" refers to a protein that differs from the amino acid sequence of a parent ZEN-degrading enzyme in at least one amino acid and has ZEN-degrading activity.

The "variant polypeptide having ZEN-degrading activity" may be used interchangeably with "variant of a parent ZEN-degrading enzyme" and "ZEN-degrading enzyme variant".

The variant provided herein may be one that has ZEN-degrading activity and comprises the modification of at least one amino acid in the sequence of a parent ZEN-degrading enzyme.

Additionally, the variant may be: i) a polypeptide having a sequence identity of at least 70% and less than 100% to SEQ ID NO: 1; and/or ii) a polypeptide encoded by a polynucleotide having a sequence identity of at least 70% and less than 100% to a sequence encoding the mature polypeptide of SEQ ID NO: 1; and/or iii) a polypeptide encoded by a polynucleotide that hybridizes under low stringency conditions, medium stringency conditions, medium-high stringency conditions, high stringency conditions, or very high stringency conditions with (a) the mature polypeptide coding sequence of SEQ ID NO: 1, (b) a cDNA thereof, or (c) a full-length complement of (a) or (b); and/or iv) a functional fragment of i), ii), or iii) having ZEN-degrading activity, and the variant may comprise the substitution of at least one amino acid at positions 22, 43, 45, and 111 with a different amino acid,.

Specifically, the variant provided herein may have ZEN-degrading activity and comprise the modification of at least one amino acid in the sequence of a parent ZEN-degrading enzyme, thereby exhibiting at least one altered function or property compared to the parent ZEN-degrading enzyme.

In one specific embodiment, the variant provided herein may have ZEN-degrading activity and comprise the modification of at least one amino acid in the sequence of a parent ZEN-degrading enzyme, thereby exhibiting at least one altered function or property compared to the parent ZEN-degrading enzyme, and may have at least one conserved substitution.

The variant provided herein is a variant of a parent ZEN-degrading enzyme and may be a polypeptide having ZEN-degrading enzyme activity.

In one specific embodiment, the variant provided herein may comprise a modification at at least one position selected from the amino acids corresponding to positions 22, 43, 45, and 111 of SEQ ID NO: 1.

As used herein, the position numbers correspond to the positions in the polypeptide of SEQ ID NO: 1, and the term "corresponding" is as described above.

In one specific embodiment, the variant provided herein may comprise the modification of at least one amino acid selected from the amino acids corresponding to P22, K43, A45, and T111 of SEQ ID NO: 1.

In one specific embodiment, before the modification provided in the present disclosure, the amino acid corresponding to position 22 of SEQ ID NO: 1 may be proline (P); the amino acid corresponding to position 43 may be lysine (K); the amino acid corresponding to position 45 may be alanine (A); and/or the amino acid corresponding to position 111 may be threonine (T).

In one specific embodiment, the variant provided in the present disclosure may comprise a substitution of P, which is the amino acid corresponding to position 22 of SEQ ID NO: 1, with G, V, L, I, M, F, W, S, T, C, Y, N, Q, D, E, K, R, or H; as one example, may comprise a substitution with S, T, C, Y, N, or Q; as another example, may comprise a substitution with S, T, N, or Q; and as yet another example, may comprise a substitution with T.

In one specific embodiment, the variant provided in the present disclosure may comprise a substitution of K, which is the amino acid corresponding to position 43 of SEQ ID NO: 1, with G, A, L, I, M, F, W, P, S, T, C, Y, N, Q, D, E, R, or H; as one example, may comprise a substitution with G, A, V, L, I, M, F, W or P; as another example, may comprise a substitution with G, A, V, L, I, or M ; and as yet another example, may comprise a substitution with A.

In one specific embodiment, the variant provided in the present disclosure may comprise a substitution of A, which is the amino acid corresponding to position 45 of SEQ ID NO: 1, with G, V, I, M, F, W, P, S, T, C, Y, N, Q, D, E, K, R, or H; as one example, may comprise a substitution with S, T, C, Y, N, or Q; as another example, may comprise a substitution with S, T, C, N, or Q; and as yet another example, may comprise a substitution with C.

In one specific embodiment, the variant provided in the present disclosure may comprise a substitution of T, which is the amino acid corresponding to position 111 of SEQ ID NO: 1, with G, A, V, L, I, F, W, P, S, C, Y, N, Q, D, E, K, R, or H; as one example, may comprise a substitution with S, C, Y, N, or Q; as another example, may comprise a substitution with S, C, N, or Q; and as yet another example, may comprise a substitution with C.

In one specific embodiment, the variant provided herein may comprise a substitution of the amino acid corresponding to position 22 of SEQ ID NO: 1 with a polar or hydrophilic amino acid.

In one specific embodiment, the variant provided herein may comprise a substitution of the amino acid corresponding to position 43 of SEQ ID NO: 1 with a nonpolar amino acid.

In one specific embodiment, the variant provided herein may comprise a substitution of the amino acid corresponding to position 45 of SEQ ID NO: 1 with a polar or hydrophilic amino acid.

In one specific embodiment, the variant provided herein may comprise a substitution of the amino acid corresponding to position 111 of SEQ ID NO: 1 with a polar or hydrophilic amino acid.

In one specific embodiment, the variant provided herein may comprise at least one substitution selected from P22T, K43A, A45C, and T111C of SEQ ID NO: 1. In one specific embodiment, the variant provided herein may comprise a combination of one, two, three, or four amino acid substitutions selected from the above-described amino acid substitutions.

In one embodiment, the variants provided herein comprise all possible combinations of the above-described variants.

For example, the variant may comprise amino acid modification at a position selected from i) to xv) below as a combination of the above-described modifications: i) position 22; ii) position 43; iii) position 45; iv) position 111; v) positions 22+43; vi) positions 22+45; vii) positions 22+111; viii) positions 43+45; ix) positions 43+111; x) positions 45+111; xi) positions 22+43+45; xii) positions 22+43+111; xiii) positions 22+45+111; xiv) positions 43+45+111; and xv) positions 22+43+45+111.

In another example, the variant may be one comprising at least one substitution selected from i) to iv) below, but is not limited thereto: i) substitution of the amino acid at position 22 with threonine; ii) substitution of the amino acid at position 43 with alanine; iii) substitution of the amino acid at position 45 with cysteine; and iv) substitution of the amino acid at position 111 with cysteine.

In another example, the variant may comprise at least one modification selected from the following, but is not limited thereto:
P22T
K43A
A45C
T111C
P22T+K43A
P22T+A45C
P22T+T111C
K43A+A45C
K43A+T111C
A45C+T111C
P22T+K43A+A45C
P22T+K43A+T111C
P22T+A45C+T111C
K43A+A45C+T111C
P22T+K43A+A45C+T111C

In one embodiment, the variant provided in the present disclosure may have a sequence identity of at least about 60%, for example, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, and less than 100%, to the parent ZEN-degrading enzyme; a mature polypeptide thereof; or a functional fragment thereof.

In one embodiment, the variant provided in the present disclosure may be one having a sequence identity of at least about 60%, for example, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, and less than 100%, to SEQ ID NO: 1.

In one embodiment, the variant provided in the present disclosure may be a polypeptide encoded by a polynucleotide having a sequence identity of at least about 60%, for example, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, and less than 100%, to the sequence encoding the mature polypeptide of SEQ ID NO: 1.

In one embodiment, the variant provided in the present disclosure may be one having a sequence identity of at least about 60%, for example, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, and less than 100%, to a functional fragment of SEQ ID NO: 1.

Specifically, provided in the present disclosure, a variant of SEQ ID NO: 1 comprising A45C and T111C substitutions may be represented by SEQ ID NO: 5, a variant of SEQ ID NO: 1 comprising K43A substitution may be represented by SEQ ID NO: 6, and a variant of SEQ ID NO: 1 comprising P22T substitution may be represented by SEQ ID NO: 7.

In addition, a variant polypeptide that differs from the recited sequence in at least one amino acid by conservative substitution and/or modification as compared to the amino acid sequence of any one of SEQ ID NOs: 5 to 7, but retains the functions or properties of the protein, also falls within the scope of the polypeptide provided in the present disclosure. Such modifications may comprise, for example, a modification in which a portion is removed from the N-terminus and/or C-terminus of the mature protein.

The polypeptide of the present disclosure may have, for example, at least one conservative substitution while retaining at least one biological activity of the polypeptide of any one of SEQ ID NOs: 5 to 7. Typically, a conserved substitution has little or no effect on the activity of the produced polypeptide. Typically, a conserved substitution may have little or no effect on the activity of a protein or polypeptide.

In one embodiment, the polypeptide provided in the present disclosure may comprise a polypeptide consisting of, comprising, or consisting essentially of the amino acid sequence of any one of SEQ ID NOs: 5 to 7 or an amino acid sequence having at least 80%, for example, at least 85%, 90%, 95%, 97%, or 99% or more homology or identity to any one of SEQ ID NOs: 5 to 7. In addition, a polypeptide having the above homology or identity and having ZEN-degrading activity falls within the scope of the polypeptide having ZEN-degrading activity provided in the present disclosure.

The variant polypeptide provided herein may exhibit alterations in at least one selectable or detectable property or attribute of a polypeptide as compared to other parent ZEN-degrading enzymes, such as wild-type ZEN-degrading enzymes, parent ZEN-degrading enzymes, and other variant polypeptides having ZEN-degrading activity.

The properties or attributes comprise oxidative stability, substrate specificity, catalytic activity, thermal stability, acid stability, alkaline stability, pH activity profile, resistance to proteolytic degradation, Km, k_{cat}, k_{cat}/Km ratio, protein folding, immune response induction, ability to bind to a ligand, ability to bind to a receptor, ability to be secreted, ability to be displayed on the surface of a cell, ability to oligomerize, ability to send a signal, ability to stimulate cell proliferation, ability to inhibit cell proliferation, ability to induce apoptosis, ability to be modified by phosphorylation or glycosylation, and/or ability to treat diseases, *etc.*, but are not limited thereto.

Specifically, the variant provided herein may have pH stability or exhibit activity with increased pH stability compared to a parent sequence.

Enzyme stability means that enzymatic activity is retained during storage or reaction time. In order to measure such changes in stability, the initial enzymatic activity can be measured and compared under defined conditions at time zero (100%) and after a certain period of time (x%), and thus, the level at which the enzymatic activity is lost or enzyme stability can be expressed. Factors that affect enzymatic activity comprise, for example, pH, heat, the presence of other substances (*e.g.*, oxidizing agents, chelating agents), *etc.*

As used herein, the term "pH stability" refers to the ability of a protein to function within a specific pH range. In one embodiment, the variant provided herein may be active at about pH 2.0 to about pH 12.0, but is not limited thereto.

If a protein maintains its function within a specific pH range, it can be defined as having "pH stability", and depending on the pH range, it can be defined as having "acid tolerance (acid stability)", "alkali tolerance", *etc.*

The increase in stability comprises maintenance of high enzymatic activity and an increased range of pH over which the protein maintains its function, compared to other enzymes, for example, wild-type enzymes, parent enzymes, and/or other variants.

In one specific embodiment, a variant polypeptide in which the amino acids corresponding to positions 45 and 111 of SEQ ID NO: 1 are substituted with cysteine, a variant polypeptide in which the amino acid corresponding to position 43 of SEQ ID NO: 1 is substituted with alanine, or a variant polypeptide in which the amino acid corresponding to position 22 of SEQ ID NO: 1 is substituted with threonine may have acid tolerance or may have increased acid tolerance (acid stability) as compared to a polypeptide consisting of the amino acid sequence of SEQ ID NO: 1.

Specifically, with respect to the acid tolerance, the degradation rate at an acidic pH may be at least 50%, specifically at least 60%, and more specifically 60% to 90%, relative to the degradation rate at the optimal pH, but is not limited thereto. The optimal pH may be the optimal pH of the parent sequence or the variant, and specifically may be pH 8. The acidic pH may be pH 2 to 4, and specifically may be pH 3.

In one specific embodiment, the variant polypeptide consisting of the amino acid sequence of any one of SEQ ID NOs: 5 to 7 may have acid tolerance or may have increased acid tolerance (acid stability) as compared to a polypeptide consisting of the amino acid sequence of SEQ ID NO: 1.

In one example of the present disclosure, as a result of evaluating the acid tolerance (acid stability) of the variant polypeptide, it was confirmed that the variant polypeptide has acid tolerance and exhibits superior ZEN-degrading activity under acidic conditions as compared to the polypeptide of SEQ ID NO: 1, thereby confirming that the acid tolerance (acid stability) has been improved.

Another aspect of the present disclosure provides a polynucleotide encoding the variant polypeptide having ZEN-degrading activity of the present disclosure.

As used herein, the term "polynucleotide", which is a polymer of nucleotides in which nucleotide monomers are connected in a long chain shape by covalent bonds, is a DNA or RNA strand having a certain length or more. More specifically, it refers to a polynucleotide fragment encoding the variant.

The polynucleotide encoding the variant of the present disclosure may comprise the coding sequence of the above-described variant. The polynucleotide may undergo various modifications in the coding region within the scope that do not change the amino acid sequence of the polypeptide, taking into consideration codon degeneracy or the codons preferred in an organism in which the polypeptide is to be expressed.

Additionally, the polynucleotide of the present disclosure may comprise, without limitation, a probe that can be prepared from a known gene sequence, for example, any polynucleotide sequence that hybridizes with a sequence complementary to all or part of the nucleotide sequence under stringent conditions to encode the variant of the present disclosure.

The term "stringent conditions" refers to conditions that enable specific hybridization between polynucleotides. These conditions are specifically disclosed in the literature (*e.g.*, J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York).

For example, the stringent conditions may comprise conditions under which polynucleotides having high homology or identity, *i.e.*, polynucleotides having at least 40%, specifically at least 90%, more specifically at least 95%, at least 96%, at least 97%, or at least 98%, and even more specifically at least 99% homology or identity hybridize with each other, while polynucleotides having homology or identity lower than the above homology or identity do not hybridize with each other; or may comprise ordinary washing conditions of Southern hybridization, *i.e.*, washing once, specifically twice or three times, at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having homology or identity may be detected using hybridization conditions comprising hybridization at a Tₘ value of 55°C, as well as the above-described conditions. In addition, the Tₘ value may be 60°C, 63°C, or 65°C, but is not limited thereto, and may be appropriately adjusted by those skilled in the art depending on the purpose thereof.

The appropriate stringency for hybridizing a polynucleotide depends on the length and degree of complementarity of the polynucleotide, and the variables are well known in the relevant technical field (see J. Sambrook *et al.*, supra, 9.50-9.51, 11.7-11.8).

For example, "high stringency" occurs about 5°C to 10°C below the Tₘ of the probe; "medium stringency" occurs about 10°C to 20°C below the Tₘ of the probe; and "low stringency" may occur about 20°C to 25°C below the Tₘ, but is not limited thereto.

In one example, a "low stringency condition" may be prehybridization and hybridization at 42°C in 5X SSPE, 0.3% SDS, sheared and denatured salmon sperm DNA (200 µg/mL), and 25% formamide for 12 to 24 hours according to the Southern blotting standard procedure for probes of at least 100 nucleotides in length. The carrier material can finally be washed 2 to 3 times for 15 minutes each, using 2 X SSC and 0.1% to 0.2% SDS at 50°C.

In one example, a "medium stringency condition" may be prehybridization and hybridization at 42°C in 5X SSPE, 0.3% SDS, sheared and denatured salmon sperm DNA (200 µg/mL), and 35% formamide for 12 to 24 hours according to Southern blotting standard procedures for probes of at least 100 nucleotides in length. The carrier material can finally be washed 2 to 3 times for 15 minutes each, using 2 X SSC and 0.1% to 0.2% SDS at 55°C. In one example, a "medium-high stringency condition" may be prehybridization and hybridization at 42°C in 5X SSPE, 0.3% SDS, sheared and denatured salmon sperm DNA (200 µg/mL), and 35% formamide for 12 to 24 hours according to the Southern blotting standard procedure for probes of at least 100 nucleotides in length. The carrier material can finally be washed 2 to 3 times each for 15 minutes, using 1 to 2 X SSC and 0.1% to 0.2% SDS at 60°C.

In one example, a "high stringency condition" may be prehybridization and hybridization at 42°C in 5 X SSPE, 0.3% SDS, sheared and denatured salmon sperm DNA (200 µg/mL), and 35% formamide for 12 to 24 hours according to the Southern blotting standard procedure for probes of at least 100 nucleotides in length. The carrier material can finally be washed 2 to 3 times for 15 minutes each, using 2 X SSC and 0.1% to 0.2% SDS at 65°C.

The polynucleotide encoding the variant polypeptide having ZEN-degrading activity of the present disclosure may comprise any polynucleotide without limitation, as long as it encodes the polypeptide of any one of SEQ ID NOs: 5 to 7 and a polypeptide having the corresponding activity. For example, the polynucleotide encoding the polypeptide having ZEN-degrading activity of the present disclosure may be a polynucleotide sequence encoding the amino acid sequence of any one of SEQ ID NOs: 5 to 7 or a polypeptide having at least 80% homology or identity thereto.

In one embodiment, the polynucleotide encoding the variant polypeptide having ZEN-degrading activity of the present disclosure may consist of or consist essentially of a nucleotide sequence having at least 80%, 85%, 90%, 95%, 96%, 97%, 98%, or 99% or more homology or identity to the sequence of any one of SEQ ID NOs: 8 to 10, but is not limited thereto.

The nucleic acid construct provided herein comprises a polynucleotide encoding the variant provided in the present disclosure that is operably linked to at least one regulatory sequence that directs the expression of a coding sequence in a suitable host cell under conditions suitable for the regulatory sequences.

Polynucleotides can be manipulated in various ways to enable the expression of the variant. Depending on the expression vector, it may be desirable or necessary to manipulate the polynucleotide before inserting the polynucleotide into the vector. Such manipulation may use methods known in the art.

As used herein, the term "transformation" refers to the introduction of a vector comprising a polynucleotide encoding a target protein into a host cell to enable the expression of the protein encoded by the polynucleotide within the host cell. As long as the transformed polynucleotide can be expressed within the host cell, the transformed polynucleotide may be located within the chromosome of the host cell or exist extrachromosomally. In addition, the polynucleotide comprises DNA and RNA encoding the target protein. The polynucleotide may be introduced in any form, provided that it can be introduced into and expressed within a host cell. For example, the polynucleotide may be introduced into a host cell in the form of an expression cassette, which is a gene construct comprising all elements necessary for self-expression. The expression cassette may comprise a promoter operably linked to the polynucleotide, a transcription termination signal, a ribosome binding site, and a translation termination signal. The expression cassette may be in the form of a self-replicating expression vector. In addition, the polynucleotide may be introduced into a host cell as-is and operably linked to a sequence necessary for expression in the host cell, but is not limited thereto.

The method for transforming the vector of the present disclosure includes any method of introducing a nucleic acid into a cell and may be performed by selecting a suitable standard technique as known in the art, depending on the host cell. For example, electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method may be used, but the method is not limited thereto.

The "vector" provided herein refers to a DNA product containing the base sequence of a polynucleotide encoding the variant, which is operably linked to a suitable expression control region (or expression control sequence) so as to enable the expression of the variant in a suitable host. The expression regulatory sequence may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector that can be used in the present disclosure is not particularly limited, and any vector known in the art may be used. Examples of commonly used vectors comprise plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.*, may be used; and as a plasmid vector, those based on pBR, pUC, pBluescriptII, pGEM, pTZ, pCL, pET, *etc.*, may be used. Specifically, pDZ, pACYC177, pACYC184, pCL, pECCG117, pUC19, pBR322, pMW118, pCC1BAC vectors, and the like may be used.

In an example, a polynucleotide encoding the variant provided herein may be inserted into a chromosome through a vector for chromosomal insertion in a cell. The insertion of a polynucleotide into the chromosome may be performed by any method known in the art, such as homologous recombination, but is not limited thereto. A selection marker for confirming the chromosomal insertion may be further comprised. The selection marker is used to screen cells transformed with the vector, *i.e.*, to confirm the insertion of a desired nucleic acid molecule; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a different phenotype, allowing for the selection of transformed cells.

The host cell of the present disclosure may comprise any cell without limitation, as long as it is capable of expressing the variant of the present disclosure.

The host cell of the present disclosure may comprise the above-described variant, a polynucleotide encoding the variant, a nucleic acid construct, and/or a vector comprising the same.

The nucleic acid construct or vector may be incorporated into a chromosome as described above, or maintained as a self-replicating extrachromosomal vector.

The host cell of the present disclosure comprises any progeny of a parent cell that is not identical to the parent cell due to mutations occurring during replication.

The host cell can be any cell, for example, a prokaryotic or eukaryotic cell, useful for generating the recombination of the variant.

The prokaryotic host cell may be any Gram-positive or Gram-negative bacterium.

The Gram-positive bacterium comprises *Bacillus, Clostridium, Enterococcus, Geobacillus, Lactobacillus, Lactococcus, Oceanobacillus, Staphylococcus, Streptococcus,* and *Streptomyces,* but is not limited thereto.

The Gram-negative bacterium comprises *Campylobacter, Escherichia coli* (*E. coli), Flavobacterium, Fusobacterium, Helicobacter, Illiobacter, Neisseria, Pseudomonas, Salmonella, Vibrio* (*e.g., Vibrio natriegens*), and *Ureaplasma,* but is not limited thereto.

In one embodiment, the bacterial host cell may be a host cell of the genus *Bacillus,* and specifically, it comprises *Bacillus alcalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis,* but is not limited thereto.

In one embodiment, the bacterial host cell may be a host cell of the genus *Streptococcus,* and specifically, it comprises *Streptococcus equisimilis, Streptococcus pyogenes, Streptococcus uberis,* and *Streptococcus equi subsp. zooepidemicus,* but is not limited thereto.

In one embodiment, the bacterial host cell may be a host cell of the genus *Streptomyces,* and specifically, it comprises *Streptomyces achromogenes, Streptomyces avermitilis, Streptomyces coelicolor, Streptomyces griseus,* and *Streptomyces lividans,* but is not limited thereto.

In one embodiment, the bacterial host cell may be a host cell of the genus *Corynebacterium,* and may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

The host cell may be a eukaryote, such as a mammal, insect, plant, or fungal cell.

The host cell may be a fungal cell. In the present disclosure, "fungus" comprises *Ascomycota, Basidiomycota, Chytridiomycota,* and *Zygomycota,* as well as *Oomycota* and all imperfect fungi.

Fungal host cells may be yeast cells. In the present disclosure, "yeast" comprises ascosporogenous yeasts (*Endomycetales*), basidiosporogenous yeasts, and yeasts belonging to the Fungi imperfecti (*Blastomycetes*)*.* However, this classification may change and may be defined as described in Biology and Activities of Yeast (Skinner, Passmore, and Davenport, editors, Soc. App. Bacteriol. Symposium Series No. 9, 1980).

Yeast host cells may be a *Candida, Hansenula, Kluyveromyces, Pichia, Komagatella, Saccharomyces, Schizosaccharomyces,* or *Yarrowia* cell, for example, a *Kluyveromyces lactis, Saccharomyces carlsbergensis, Saccharomyces cerevisiae, Saccharomyces diastaticus, Saccharomyces douglasii, Saccharomyces kluyveri, Saccharomyces norvensis, Saccharomyces orbensis, Saccharomyces oviformis, Komagatella phaffii,* or *Yarrowia lipolytica* cell.

The fungal host cell may be a filamentous fungal cell. "Filamentous fungi" comprises all filamentous forms of the *Mycophylum* and *Oomycota* subphylum (as defined in the literature (Hawksworth *et al.,* 1995)). Filamentous fungi are generally characterized by hyphae walls consisting of chitin, cellulose, glucan, chitosan, mannan, and other complex polysaccharides. Vegetative growth is by hyphae elongation, and carbon catabolism is absolutely aerobic. In contrast, vegetative growth by yeast, for example, *Saccharomyces cerevisiae,* is by the germination of a unicellular thallus, and carbon catabolism may be fermentable.

Filamentous fungal host cells comprise an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may comprise an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell. However, it is not limited thereto.

In one example of the present disclosure, *Escherichia coli* (*E. coli*) was used as the host cell, but is not limited thereto.

The host cell expressing the variant polypeptide of the present disclosure may be a host cell comprising at least one selected from the variant polypeptide of the present disclosure, a polynucleotide encoding the polypeptide, and a vector comprising the polynucleotide.

Still another aspect of the present disclosure provides a composition comprising at least one selected from the variant polypeptide of the present disclosure, a polynucleotide encoding the polypeptide, a vector comprising the polynucleotide, and a host cell expressing the polypeptide.

The composition of the present disclosure may be used to convert a substrate of the ZEN-degrading enzyme into a final product, and may be a composition for degrading ZEN, a composition for detoxifying ZEN present in food and/or feed, or a composition for use as a feed additive, but is not limited thereto.

The variant polypeptide of the present disclosure, a polynucleotide encoding the polypeptide, a vector comprising the polynucleotide, and/or a host cell expressing the polypeptide may be used to degrade zearalenone.

The terms used herein are as described above, and the degradation of ZEN may be used to mean the detoxification of ZEN, the inactivation of ZEN, and the decontamination of contamination caused by ZEN.

In one embodiment, ZEN may be present in food.

In one embodiment, ZEN may be present in feed.

In one embodiment, ZEN present in food and/or feed can be degraded and detoxified using the composition of the present disclosure.

The composition of the present disclosure may further comprise other components in addition to the variant provided herein. Those skilled in the art can appropriately select components to be added to the composition of the present disclosure.

In one specific embodiment, the composition of the present disclosure may further comprise any component suitable for application in converting a substrate of the ZEN-degrading enzyme into a final product.

Examples of substances that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, *etc.*, but are not limited thereto.

In one embodiment, the composition provided herein may further comprise a naturally occurring substance or a non-naturally occurring substance in addition to the variant provided herein.

In one embodiment, the composition provided herein may further comprise additional enzymes used in ZEN degradation in addition to the variant provided herein.

In one embodiment, the vector may be incorporated into a chromosome as described above, or maintained as a self-replicating extrachromosomal vector.

In the composition of the present disclosure, the variant polypeptide may be recovered from a microorganism expressing the polypeptide, or a host cell itself expressing the polypeptide may be used as a source of the polypeptide without recovering the polypeptide.

Still another aspect of the present disclosure provides a method for degrading zearalenone, comprising contacting zearalenone with at least one selected from the variant polypeptide of the present disclosure, a polynucleotide encoding the polypeptide, a vector comprising the polynucleotide, and a host cell expressing the polypeptide.

The terms used herein are as described above.

In the method of the present disclosure, the variant polypeptide may be recovered from a microorganism expressing the polypeptide, or a host cell itself expressing the polypeptide may be used as a source of the polypeptide without recovering the polypeptide.

The method for producing a variant of the present disclosure may comprise: culturing a host cell; and recovering a variant expressed during the culturing.

As used herein, the term "culturing" means that the host cell is grown under appropriately controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required to culture the host cell as a main component, and the medium supplies nutrients, growth factors, *etc.*, comprising water, which are indispensable for survival and development. Specifically, any medium and culture conditions may be used for culturing the host cell without particular limitation, as long as the medium is used for the common culture of host cells. The host cell may be cultured in a common medium containing suitable carbon sources, nitrogen sources, phosphorus sources, inorganic compounds, amino acids, and/or vitamins, *etc.*, while controlling the temperature, pH, *etc.*, under aerobic conditions.

In the present disclosure, the carbon sources comprise carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamate, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.*, molasses converted into reducing sugars) may be used, and other various carbon sources in appropriate amounts may be used without limitation. These carbon sources may be used alone or in combination of at least two, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamate, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of at least two, but are not limited thereto.

As the phosphorus sources, monobasic potassium phosphate, dibasic potassium phosphate, or the corresponding sodium-containing salts may be used. As for inorganic compounds, sodium chloride, calcium chloride, iron chloride, magnesium sulfate, iron sulfate, manganese sulfate, calcium carbonate, *etc.*, may be used, and in addition, amino acids, vitamins, and/or suitable precursors may be comprised. These components or precursors may be added to the medium in a batchwise or continuous manner. However, the medium is not limited thereto.

During the culturing of the host cell, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto

The temperature during the culturing of the present disclosure may be in the range from 20°C to 55°C, specifically from 25°C to 40°C, but is not limited thereto. The culturing may be continued until a desired amount of useful material is obtained, specifically for 24 hours to 196 hours, but is not limited thereto.

In one specific embodiment, the variant expressed during the culturing can be recovered using a method known in the art to which the present disclosure pertains. For example, the variant can be recovered from the nutrient medium by conventional procedures comprising, but not limited to, collection, centrifugation, filtration, extraction, spray-drying, evaporation, or precipitation.

The method of recovery may be collecting the desired variant using a suitable method known in the art, depending on the method for culturing the host cell of the present disclosure, such as batch, continuous, or fed-batch culturing. For example, the variant can be recovered from the medium or the host cell using suitable methods known in the art, such as centrifugation, filtration, crystallization, treatment with protein precipitants (salting-out method), extraction, ultrasonic disruption, ultrafiltration, dialysis, various types of chromatography such as molecular sieve chromatography (gel filtration), adsorption chromatography, ion exchange chromatography, and affinity chromatography, HPLC, and a combination thereof.

In another specific embodiment, the variant expressed by the host cell during the culturing may not be recovered. In the above specific embodiment, the host cell expressing the variant can be used as a source of the variant.

Still another aspect of the present disclosure provides the use of the variant polypeptide of the present disclosure as a ZEN-degrading enzyme.

The terms used herein are as described above.

Still another aspect of the present disclosure provides the use of a composition comprising at least one selected from the variant polypeptide of the present disclosure and a host cell comprising the variant polypeptide, for degrading ZEN.

The terms used herein are as described above.

### [Modes for Carrying Out the Invention]

The present disclosure will be described in detail with reference to specific embodiments as follows. Meanwhile, each description and embodiment described herein may be applied to other descriptions and embodiments, respectively. That is, all combinations of various elements disclosed herein fall within the scope of the present disclosure. In addition, the scope of the present disclosure is not limited to the specific description described below.

Furthermore, those skilled in the art may recognize or identify numerous equivalents to the specific aspects of the present disclosure described herein by using routine experimentation. Moreover, these equivalents are intended to be comprised in the present disclosure.

### Example 1. Preparation of expression vectors for ZEN-degrading enzyme ZOR_7 and variants thereof

### Example 1-1. Preparation of an expression vector for ZEN-degrading enzyme ZOR_7

A polynucleotide (SEQ ID NO: 2) encoding an alpha/beta hydrolase derived from *Sphingomonas* sp. (hereinafter referred to as ZOR_7; SEQ ID NO: 1) was synthesized by Cosmo Genetech and cloned into a pET vector (Novagen). This was used as a template, and the hydrolase sequence, the polynucleotide sequence thereof, and the primer sequences used are shown in Tables 1 and 2 below.

**[Table 1]**

| SEQ ID NO | Name | Sequence |
|---|---|---|
| 1 | ZOR_7_AA | MTSETGAPHPAHRNIFVSHSFPEQQVDLGEVTLNYVEAGD AGKPALLLVPEQTGSWWSYEPSIAMLAENFHVFAVDMRGQ GRSDWTPRRYSLDNFGNDLVRFIALVIKRPTIVAGNSSGGV LAAWLSAYAMPGQIRGALCEDAPFFASELTPAYGHPIRQAA GPAFALMRDYLGDQWSVANWEGFAAAAKASSSPIARLFFA TEEAPQNLKEYDPEWGRAFYEGTVALHCPHDRMLAQVKK PMLLTHHAHHIDPETGDLVGALSDFQAQKVQEIVRSTGVRI DYQSFPDALHMMHLFDPALYTQVLREWAATLPDA |
| 2 | ZOR_7_NT | ATGACGAGCGAAACCGGCGCGCCGCATCCGGCGCATCG CAACATTTTTGTGAGCCATAGCTTTCCGGAACAGCAAGT GGATCTGGGCGAAGTGACCCTGAACTATGTGGAAGCGG GCGATGCGGGCAAACCGGCGCTGCTGCTGGTGCCGGAA CAGACCGGCAGCTGGTGGAGCTATGAACCGAGCATTGC GATGCTGGCGGAAAACTTTCATGTGTTTGCGGTGGATAT GCGCGGCCAAGGCCGCAGCGATTGGACCCCGCGCCGC TATAGCCTGGATAACTTTGGCAACGATCTGGTGCGCTTT ATTGCGCTGGTGATTAAGCGCCCTACCATCGTGGCTGGT AACAGCAGCGGGGGTGTCTTGGCTGCGTGGCTGAGCGC GTATGCGATGCCGGGTCAGATTCGCGGCGCGCTGTGCG AAGATGCGCCGTTTTTTGCGAGCGAACTGACCCCGGCGT |
| | | ATGGCCATCCGATTCGCCAAGCGGCGGGCCCGGCGTTT GCGCTGATGCGCGATTATCTGGGCGATCAGTGGAGCGT GGCGAACTGGGAAGGCTTTGCGGCGGCGGCGAAAGCG AGCAGCAGCCCGATTGCGCGCCTGTTTTTTGCGACCGAA GAAGCGCCGCAGAACCTGAAAGAATATGATCCGGAATG GGGCCGCGCGTTTTATGAAGGCACCGTGGCGCTGCATT GCCCGCATGATCGCATGCTGGCGCAAGTGAAAAAACCG ATGCTGCTGACCCATCATGCGCATCATATTGATCCGGAA ACCGGCGATCTGGTGGGCGCGCTGAGCGATTTTCAAGC GCAGAAAGTGCAAGAAATTGTGCGCAGCACCGGCGTGC GCATTGATTATCAGAGCTTTCCGGATGCGCTGCACATGA TGCATCTGTTTGATCCGGCGCTGTATACCCAAGTGCTGC GCGAATGGGCGGCGACCCTGCCGGATGCG |

**[Table 2]**

| Sequence | SEQ ID NO | Name | Primer sequence (5'→3') |
|---|---|---|---|
| ZOR7 | 3 | ZOR7-F | gatataccatggggATGACGAGCGAAACCGGCG |
| | 4 | ZOR7-R | cgagtgcggccgcCGCATCCGGCAGGGTCG |

Specifically, ZOR_7 was prepared using the synthesized gene construct, primers (SEQ ID Nos: 3 and 4 in Table 2), and PCR premix (iNtRON, Cat. No. 25185). PCR was performed using an Eppendorf Mastercycler Nexus GX2, and the reaction conditions are as follows.
Initial denaturation - 94°C, 2 minutes
Denaturation - 94°C, 20 seconds
Annealing - 55°C, 10 seconds
Extension - 72°C, 2 minutes (25 cycles of denaturation to extension)
Final extension - 72°C, 5 minutes

The thus-formed PCR product and the vector were treated with restriction enzymes (NcoI and NotI), ligated using T4 DNA ligase (NEB, Cat# M0202S), transformed into *E. coli* DH5α, and confirmed for sequence mutation through sequencing.

### Example 1-2. Preparation of expression vectors for variants of the ZEN-degrading enzyme ZOR_7

Mutation sites were selected to improve the acid stability of ZEN-degrading enzyme ZOR_7, and primers were designed to prepare three variants with point mutation or multiple mutations (hereinafter referred to as ZOR7_M1, ZOR7_M2, and ZOR7_M3; SEQ ID NOs: 5, 6, and 7). The sequence mutation positions refer to the mutation positions based on the sequence of SEQ ID NO: 1, and the amino acid sequences of the variants, the polynucleotide sequences thereof, and the primer sequences for preparing the variants are set forth in Table 3 and Table 4 below.

**[Table 3]**

| SEQ ID NO | Name | Sequence |
|---|---|---|
| 5 | ZOR7_M1 AA(A45C+T 111C) | MTSETGAPHPAHRNIFVSHSFPEQQVDLGEVTLNYVEAGDA GKPCLLLVPEQTGSWWSYEPSIAMLAENFHVFAVDMRGQGR SDWTPRRYSLDNFGNDLVRFIALVIKRPCIVAGNSSGGVLAA WLSAYAMPGQIRGALCEDAPFFASELTPAYGHPIRQAAGPAF ALMRDYLGDQWSVANWEGFAAAAKASSSPIARLFFATEEAP QNLKEYDPEWGRAFYEGTVALHCPHDRMLAQVKKPMLLTHH AHHIDPETGDLVGALSDFQAQKVQEIVRSTGVRIDYQSFPDAL HMMHLFDPALYTQVLREWAATLPDA |
| 6 | ZOR7_M2 AA(K43A) | MTSETGAPHPAHRNIFVSHSFPEQQVDLGEVTLNYVEAGDA GAPALLLVPEQTGSWWSYEPSIAMLAENFHVFAVDMRGQGR SDWTPRRYSLDNFGNDLVRFIALVIKRPTIVAGNSSGGVLAA WLSAYAMPGQIRGALCEDAPFFASELTPAYGHPIRQAAGPAF ALMRDYLGDQWSVANWEGFAAAAKASSSPIARLFFATEEAP QNLKEYDPEWGRAFYEGTVALHCPHDRMLAQVKKPMLLTHH AHHIDPETGDLVGALSDFQAQKVQEIVRSTGVRIDYQSFPDAL HMMHLFDPALYTQVLREWAATLPDA |
| 7 | ZOR7_M3 AA (P22T) | MTSETGAPHPAHRNIFVSHSFTEQQVDLGEVTLNYVEAGDAG |
| | | KPALLLVPEQTGSWWSYEPSIAMLAENFHVFAVDMRGQGRS DWTPRRYSLDNFGNDLVRFIALVIKRPTIVAGNSSGGVLAAWL SAYAMPGQIRGALCEDAPFFASELTPAYGHPIRQAAGPAFAL MRDYLGDQWSVANWEGFAAAAKASSSPIARLFFATEEAPQN LKEYDPEWGRAFYEGTVALHCPHDRMLAQVKKPMLLTHHAH HIDPETGDLVGALSDFQAQKVQEIVRSTGVRIDYQSFPDALH MMHLFDPALYTQVLREWAATLPDA |
| 8 | ZOR7_M1 NT(A45C+T 111C) | ATGACGAGCGAAACCGGCGCGCCGCATCCGGCGCATCGC AACATTTTTGTGAGCCATAGCTTTCCGGAACAGCAAGTGGA TCTGGGCGAAGTGACCCTGAACTATGTGGAAGCGGGCGAT GCGGGCAAACCGtgtCTGCTGCTGGTGCCGGAACAGACCG GCAGCTGGTGGAGCTATGAACCGAGCATTGCGATGCTGG CGGAAAACTTTCATGTGTTTGCGGTGGATATGCGCGGCCA AGGCCGCAGCGATTGGACCCCGCGCCGCTATAGCCTGGA TAACTTTGGCAACGATCTGGTGCGCTTTATTGCGCTGGTGA TTAAGCGCCCTtgtATCGTGGCTGGTAACAGCAGCGGGGGT GTCTTGGCTGCGTGGCTGAGCGCGTATGCGATGCCGGGT CAGATTCGCGGCGCGCTGTGCGAAGATGCGCCGTTTTTG CGAGCGAACTGACCCCGGCGTATGGCCATCCGATTCGCC AAGCGGCGGGCCCGGCGTTTGCGCTGATGCGCGATTATC TGGGCGATCAGTGGAGCGTGGCGAACTGGGAAGGCTTTG CGGCGGCGGCGAAAGCGAGCAGCAGCCCGATTGCGCGC CTGTTTTTTGCGACCGAAGAAGCGCCGCAGAACCTGAAAG AATATGATCCGGAATGGGGCCGCGCGTTTTATGAAGGCAC CGTGGCGCTGCATTGCCCGCATGATCGCATGCTGGCGCA AGTGAAAAAACCGATGCTGCTGACCCATCATGCGCATCAT ATTGATCCGGAAACCGGCGATCTGGTGGGCGCGCTGAGC GATTTTCAAGCGCAGAAAGTGCAAGAAATTGTGCGCAGCA CCGGCGTGCGCATTGATTATCAGAGCTTTCCGGATGCGCT GCACATGATGCATCTGTTTGATCCGGCGCTGTATACCCAA GTGCTGCGCGAATGGGCGGCGACCCTGCCGGATGCG |
| 9 | ZOR7_M2 NT(K43A) | ATGACGAGCGAAACCGGCGCCGCATCCGGCGCATCGC AACATTTTTGTGAGCCATAGCTTTCCGGAACAGCAAGTGGA |
| | | TCTGGGCGAAGTGACCCTGAACTATGTGGAAGCGGGCGAT GCGGGCgcgCCGGCGCTGCTGCTGGTGCCGGAACAGACC GGCAGCTGGTGGAGCTATGAACCGAGCATTGCGATGCTG GCGGAAAACTTTCATGTGTTTGCGGTGGATATGCGCGGCC AAGGCCGCAGCGATTGGACCCCGCGCCGCTATAGCCTGG ATAACTTTGGCAACGATCTGGTGCGCTTTATTGCGCTGGTG ATTAAGCGCCCTACCATCGTGGCTGGTAACAGCAGCGGGG GTGTCTTGGCTGCGTGGCTGAGCGCGTATGCGATGCCGG GTCAGATTCGCGGCGCGCTGTGCGAAGATGCGCCGTTTTT TGCGAGCGAACTGACCCCGGCGTATGGCCATCCGATTCG CCAAGCGGCGGGCCCGGCGTTTGCGCTGATGCGCGATTA TCTGGGCGATCAGTGGAGCGTGGCGAACTGGGAAGGCTT TGCGGCGGCGGCGAAAGCGAGCAGCAGCCCGATTGCGC GCCTGTTTTTTGCGACCGAAGAAGCGCCGCAGAACCTGAA AGAATATGATCCGGAATGGGGCCGCGCGTTTTATGAAGGC ACCGTGGCGCTGCATTGCCCGCATGATCGCATGCTGGCG CAAGTGAAAAAACCGATGCTGCTGACCCATCATGCGCATC ATATTGATCCGGAAACCGGCGATCTGGTGGGCGCGCTGA GCGATTTTCAAGCGCAGAAAGTGCAAGAAATTGTGCGCAG CACCGGCGTGCGCATTGATTATCAGAGCTTTCCGGATGCG CTGCACATGATGCATCTGTTTGATCCGGCGCTGTATACCCA AGTGCTGCGCGAATGGGCGGCGACCCTGCCGGATGCG |
| 10 | ZOR7_M3 NT(P22T) | ATGACGAGCGAAACCGGCGCGCCGCATCCGGCGCATCGC AACATTTTTGTGAGCCATAGCTTTaccGAACAGCAAGTGGAT CTGGGCGAAGTGACCCTGAACTATGTGGAAGCGGGCGAT GCGGGCAAACCGGCGCTGCTGCTGGTGCCGGAACAGACC GGCAGCTGGTGGAGCTATGAACCGAGCATTGCGATGCTG GCGGAAAACTTTCATGTGTTTGCGGTGGATATGCGCGGCC AAGGCCGCAGCGATTGGACCCCGCGCCGCTATAGCCTGG ATAACTTTGGCAACGATCTGGTGCGCTTTATTGCGCTGGTG ATTAAGCGCCCTACCATCGTGGCTGGTAACAGCAGCGGGG GTGTCTTGGCTGCGTGGCTGAGCGCGTATGCGATGCCGG GTCAGATTCGCGGCGCGCTGTGCGAAGATGCGCCGTTTTT |
| | | TGCGAGCGAACTGACCCCGGCGTATGGCCATCCGATTCG CCAAGCGGCGGGCCCGGCGTTTGCGCTGATGCGCGATTA TCTGGGCGATCAGTGGAGCGTGGCGAACTGGGAAGGCTT TGCGGCGGCGGCGAAAGCGAGCAGCAGCCCGATTGCGC GCCTGTTTTTTGCGACCGAAGAAGCGCCGCAGAACCTGAA AGAATATGATCCGGAATGGGGCCGCGCGTTTTATGAAGGC ACCGTGGCGCTGCATTGCCCGCATGATCGCATGCTGGCG CAAGTGAAAAAACCGATGCTGCTGACCCATCATGCGCATC ATATTGATCCGGAAACCGGCGATCTGGTGGGCGCGCTGA GCGATTTTCAAGCGCAGAAAGTGCAAGAAATTGTGCGCAG CACCGGCGTGCGCATTGATTATCAGAGCTTTCCGGATGCG CTGCACATGATGCATCTGTTTGATCCGGCGCTGTATACCCA AGTGCTGCGCGAATGGGCGGCGACCCTGCCGGATGCG |

**[Table 4]**

| Variant Name | Mutation | | SEQ ID NO | Primer sequence (5'→3') |
|---|---|---|---|---|
| ZOR7_ M1 | A45C | Forward | 11 | GATGCGGGCAAACCGtgtCTGCTGC TGGTGCCG |
| | | Reverse | 12 | acaCGGTTTGCCCGCATCGCC |
| | T111C | Forward | 13 | GTGATTAAGCGCCCTtgtATCGTGG CTGGTAAC |
| | | Reverse | 14 | acaAGGGCGCTTAATCACCAG |
| ZOR7_ M2 | K43A | Forward | 15 | GCGGGCGATGCGGGCgcgCCGGC GCTGCTGCTG |
| | | Reverse | 16 | cgcGCCCGCATCGCCCGCTTCCAC |
| ZOR7_ M3 | P22T | Forward | 17 | GTGAGCCATAGCTTTaccGAACAGC AAGTGGAT |
| | | Reverse | 18 | ggtAAAGCTATGGCTCACAAAAATG TTGCG |

Specifically, the three ZOR_7 variants were prepared by PCR using the template constructed in Example 1-1, primers (SEQ ID NOs: 11 to 18 in Table 4), and PCR premix (iNtRON, Cat. No. 25185). PCR was performed using an Eppendorf Mastercycler Nexus GX2, and the reaction conditions are as follows.
Initial denaturation - 94°C, 2 minutes
Denaturation - 94°C, 20 seconds
Annealing - 55°C, 10 seconds
Extension - 72°C, 2 minutes (25 cycles of denaturation to extension)
Final extension - 72°C, 5 minutes

The generated truncated variants were ligated using a QuickChange Site-Directed Mutagenesis kit (Agilent, Cat# 200518), and then transformed into *E. coli* DH5α strain to confirm sequence mutations through sequencing.

### Example 2. Expression and purification of ZEN-degrading enzyme ZOR_7 and variants thereof

The vectors of ZOR_7 and the three variants prepared in Example 1 were transformed into *E. coli* BL21(DE3), inoculated into sterilized LB medium (BD Difco), and pre-cultured at 37°C and 200 rpm for 16 hours. Thereafter, 1/100 of the culture volume was inoculated into a flask containing sterile LB medium and incubated at 37°C and 200 rpm to an OD₆₀₀ of 0.4 to 0.5. Isopropyl β-D-1-thiogalactopyranoside (IPTG) was then added to a final concentration of 1 mM, cultured for an additional 16 hours, and the cells were recovered by centrifugation. The recovered cells were added to and resuspended in 20 mL of lysis buffer (50 mM Tris-HCl, pH 8.0, 100 mM NaCl, 10 mM imidazole), and a crude enzyme solution was obtained by sonication and centrifugation. The crude enzyme solution was slowly poured onto Ni-NTA resin (Qiagen, Cat. No. 30230) for adsorption, which was then washed with wash buffer (same as lysis buffer except 20 mM imidazole) and eluted with elution buffer (same as lysis buffer except 250 mM imidazole) to purify the enzyme. The purified enzyme solution was placed in an Amicon Ultra-15 Centrifugal Filter Unit (UFC901024, Merck) and concentrated by centrifugation at 4°C, 4,000 rpm for 20 minutes. Subsequently, 50 mM Tris-HCl pH 7.4 buffer was added to the concentrated solution, and buffer exchange was performed by repeating centrifugation 2 to 3 times, and the final concentrated solution was used as the enzyme solution.

Protein concentration was determined by mixing 5 µL of the diluted enzyme solution with 250 µL of Bradford solution (Quick Start^{™} Bradford 1× Dye Reagent, #5000205) and measuring the absorbance at 595 nm.

### Example 3. Evaluation of activities of ZEN-degrading enzyme ZOR_7 and variants thereof

### Example 3-1. Evaluation of ZEN-degrading activity of ZOR_7 and variants thereof

To analyze the activity of ZOR_7 and the three variants prepared in Example 2 against zearalenone (ZEN), zearalenone (CAS 17924-92-4) dissolved in acetonitrile, and enzyme solutions were prepared, left to react at pH 8 and 37°C for 4 minutes, and the reaction was stopped by treatment with methanol. The residual amount of ZEN in the reaction mixture was measured using HPLC-UV (high performance liquid chromatography-ultraviolet), and the degrading activity of the enzymes was calculated using the ratio of the total degraded ZEN. The HPLC-UV analysis conditions are as follows.

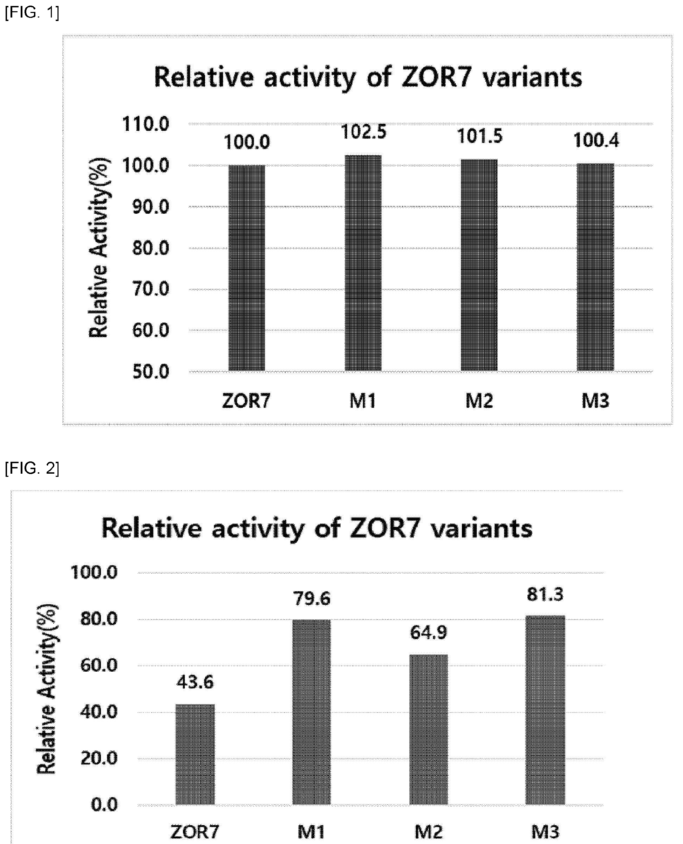

As a result, when comparing the relative activity based on the activity of ZOR_7, it was confirmed that all three variants showed a slight increase in ZEN-degrading activity (Table 5 and FIG. 1).

**[Table 5]**

| Variants | ZEN Remaining (%) | ZEN Degraded (%) | Relative Activity (%) |
|---|---|---|---|
| ZOR7 | 28.7 | 71.3 | 100.0 |
| M1 | 26.9 | 73.1 | 102.5 |
| M2 | 27.6 | 72.4 | 101.5 |
| M3 | 28.4 | 71.6 | 100.4 |

### Example 3-2. Evaluation of the acid stability of ZOR_7 and variants thereof

To evaluate the acid stability of ZOR_7 and the three variants prepared in Example 2, 2.5 µL of Glycine-HCl (1 M, pH 3.0) was mixed with 47.5 µL of the purified enzyme solution and subjected to acid treatment at 37°C for 60 minutes. Subsequently, the ZEN degradation reaction was performed for each mixture under the same reaction conditions as in Example 3-1, except for pH. The amount of ZEN degraded at pH 3 relative to the amount of ZEN degraded at pH 8 is shown in FIG. 2.

The relative activity (%) was calculated by dividing the amount of ZEN degraded at pH 3 by the amount of ZEN degraded at pH 8.

As a result of evaluating the residual enzyme activity, it was confirmed that M1, M2, and M3 all showed an improvement in residual activity of at least 20% as compared to ZOR_7 (FIG. 2).

From the above results, it was confirmed that the three variants of ZEN-degrading enzyme ZOR_7 (ZOR7_M1 of SEQ ID NO: 5, ZOR7_M2 of SEQ ID NO: 6, and ZOR7_M3 of SEQ ID NO: 7) exhibit improved acid stability as compared to the ZEN-degrading enzyme of SEQ ID NO: 1.

As set forth above, those skilled in the art will be able to understand that the present disclosure may be embodied in other specific forms without departing from the technical spirit or essential characteristics thereof. Therefore, the embodiments described above should be construed as being exemplary and not limiting the present disclosure. It should be understood that all changes or modifications derived from the definitions and the scopes of the claims and their equivalents fall within the scope of the present disclosure.

## Claims

1. A variant polypeptide having zearalenone (ZEN)-degrading activity, wherein:
i) the variant polypeptide has a sequence identity of at least 70% and less than 100% to SEQ ID NO: 1; and/or
ii) the variant polypeptide is a polypeptide encoded by a polynucleotide having a sequence identity of at least 99% and less than 100% to the sequence encoding the mature polypeptide of SEQ ID NO: 1; and/or
iii) the variant polypeptide is a polypeptide encoded by a polynucleotide that hybridizes under low stringency conditions, medium stringency conditions, medium-high stringency conditions, high stringency conditions, or very high stringency conditions with (a) the mature polypeptide coding sequence of SEQ ID NO: 1, (b) a cDNA thereof, or (c) a full-length complement of (a) or (b); and/or
iv) the variant polypeptide is a functional fragment of a polypeptide of i), ii), or iii) having zearalenone (ZEN)-degrading activity; and
the variant polypeptide comprises any one modification selected from the group consisting of:
a substitution of an amino acid at at least one position selected from positions 22, 43, 45, and 111 with a different amino acid,
wherein the position numbers correspond to positions in the polypeptide of SEQ ID NO: 1.

2. The variant polypeptide of claim 1, wherein, prior to the modification of the variant polypeptide having zearalenone (ZEN)-degrading activity, the amino acid at position 22 is proline (P); the amino acid at position 43 is lysine (K); the amino acid at position 45 is alanine (A); and the amino acid at position 111 is threonine (T).

3. The variant polypeptide of claim 1, wherein the variant polypeptide comprises a substitution of an amino acid at a position selected from i) to iii) below:
i) positions 45 and 111;
ii) position 43; and
iii) position 22,
wherein the position numbers correspond to positions in the polypeptide of SEQ ID NO: 1.

4. The variant polypeptide of claim 1, wherein the variant polypeptide comprises at least one substitution selected from i) to iii) below:
i) substitution of the amino acid at position 45 and the amino acid at position 111 with cysteine;
ii) substitution of the amino acid at position 43 with alanine; and
iii) substitution of the amino acid at position 22 with threonine,
wherein the position numbers correspond to positions in the polypeptide of SEQ ID NO: 1.

5. The variant polypeptide of claim 1, wherein the variant polypeptide has increased pH stability compared to a polypeptide consisting of the amino acid sequence of SEQ ID NO: 1.

6. A polynucleotide encoding the variant polypeptide of any one of claims 1 to 5.

7. A host cell comprising at least one selected from the variant polypeptide of any one of claims 1 to 5 and a polynucleotide encoding the variant polypeptide.

8. A composition for degrading zearalenone, comprising at least one selected from the variant polypeptide of any one of claims 1 to 5 and a host cell expressing the variant polypeptide.

9. A composition for use as a feed additive, comprising at least one selected from the variant polypeptide of any one of claims 1 to 5 and a host cell expressing the variant polypeptide.

10. A method for degrading zearalenone, comprising contacting zearalenone with at least one selected from the variant polypeptide of any one of claims 1 to 5 and a host cell expressing the variant polypeptide.

11. A method for producing a variant polypeptide having ZEN-degrading activity, comprising:
culturing a host cell comprising at least one selected from the variant polypeptide of any one of claims 1 to 5 and a polynucleotide encoding the variant polypeptide; and
recovering the variant polypeptide having ZEN-degrading activity expressed during the culturing.
